# EUROPÄISCHE PATENTSCHRIFT

(11) **EP 3 849 322 B1**
(45) Veröffentlichungstag und Bekanntmachung des Hinweises auf die Patenterteilung: **12.07.2023**
(21) Anmeldenummer: 19772975.9
(22) Anmeldetag: 11.09.2019
(51) Int. Cl.: A21C 13/02

(54) **GÄRSCHRANK ZUM GÄREN VON TEIG**
PROOFER FOR PROOFING DOUGH
ÉTUVE POUR L'ÉPREUVE DE LA PÂTE

(30) Priorität: 13.09.2018 AT 507842018
(43) Veröffentlichungstag der Anmeldung: 21.07.2021
(73) Patentinhaber: KÖNIG MASCHINEN GESELLSCHAFT M.B.H., 8045 Graz (AT)
(72) Erfinder: RIEGER, Thomas, 8501 Lieboch (AT); STELZER, Hannes, 8502 Lannach (AT)
(74) Vertreter: Wildhack & Jellinek
(86) Internationale Anmeldenummer: PCT/AT2019/060298
(87) Internationale Veröffentlichungsnummer: WO 2020/051615

(56) Entgegenhaltungen:
- EP-A1- 0 783 838
- DE-U1-202004 005 531
- JP-A- 2011 234 689
- JP-B2- 3 585 443
- US-A1- 2013 295 255

## Beschreibung

Die Erfindung betrifft einen Gärschrank.

Aus dem Stand der Technik sind Gärschränke bzw. Anlagen mit Gärschränken aus der Bäckereiindustrie bekannt, die grundsätzlich aus Blechen und Profilen aufgebaut sind. Um die Herstellung derartiger Gärschränke möglichst einfach zu halten und das Anschlagen von beispielsweise Türen und Verbindungselementen einfach durchführen zu können, werden bei derartigen Gärschränken die Profile üblicherweise waagrecht verbaut und miteinander verschraubt. Nachteilig bei den bekannten Gärschränken ist jedoch, dass diese schwierig zu reinigen sind, da im Betrieb bzw. bei der Reinigung Material wie z.B. Mehl, Schmutz und/oder Wasser auf den waagrecht verbauten Profilen zurückbleibt bzw. durch das Verschrauben der Profile Nischen für Bakterien und Verunreinigungen oder Ungeziefer entstehen. Dokument US 2013/295255 A1 offenbart einen Gärschrank nach dem Stand der Technik.

Aufgabe der Erfindung ist es daher, diese bekannten Probleme zu überwinden und einen einfach zu reinigenden Gärschrank bereitzustellen.

Die Erfindung ist ein Gärschrank gemäß Anspruch 1.

Die Erfindung löst diese Aufgabe bei einem Gärschrank zum Gären von Teig umfassend
- einen Außenrahmen, der in einem Winkel zur Schwerkraftrichtung verlaufende Rahmenprofile umfasst und einen Innenraum des Gärschranks umschließt,
- eine Anzahl an am Außenrahmen angeordneten Abdeckpaneelen,
- eine durch den Innenraum des Gärschranks verlaufende Fördervorrichtung, wobei die Fördervorrichtung dazu ausgebildet ist, Teiglinge durch den Gärschrank zu transportieren und wobei die Fördervorrichtung eine Anzahl von im Wesentlichen horizontal oder vertikal, insbesondere horizontal oder vertikal, angeordneten Transportebenen aufspannt, wobei zumindest eine Profilfläche der Rahmenprofile des Außenrahmens, die in einem Winkel zur Schwerkraftrichtung verlaufen und unterhalb derjenigen Transportebene oder desjenigen Punktes der Transportebenen angeordnet sind, die bzw. der in Schwerkraftrichtung am obersten angeordnet ist, in Richtung des Innenraums geneigt ist, mit den kennzeichnenden Merkmalen des Patentanspruchs 1.

Erfindungsgemäß ist dabei vorgesehen, dass die Rahmenprofile als Formrohre (1) ausgebildet sind und einen quadratischen oder rechteckigen Querschnitt, vier Längskanten (12) und vier Seitenflächen (11) aufweisen, wobei der Außenrahmen (10) aus normal und parallel zur Schwerkraftrichtung verlaufenden Formrohren (1) gebildet ist und zwischen der Schwerkraftrichtung und den in Richtung des Innenraums (30) geneigten Seitenflächen (11) der normal zur Schwerkraftrichtung verlaufenden Formrohre (1) ein Winkel von kleiner als 60° eingeschlossen ist.

Derart ist sichergestellt, dass im Gärschrank befindliche Verschmutzungen und Wasser in den Innenraum abfließen.

In einem Winkel zur Schwerkraftrichtung verlaufend bedeutet in diesem Zusammenhang, dass die Rahmenprofile nicht in Schwerkraftrichtung, d.h. senkrecht, verbaut sind, sondern beispielsweise in einem Winkel von kleiner oder gleich 90° zur Schwerkraftrichtung ausgerichtet sind. Durch die Neigung in Richtung des Innenraums bleibt im Betrieb bzw. bei der Reinigung des Gärschranks kein oder nur wenig Material oder Schmutz auf den Formrohren zurück, da beispielsweise Mehlreste oder Wasser, die sich im Innenraum des Gärschranks befinden, auf den Profilflächen der geneigten Rahmenprofile einfach abgleiten oder abgewaschen werden. Als Seitenflächen sind in diesem Zusammenhang diejenigen Flächen der Formrohre zu verstehen, die sich zwischen den vier Längskanten erstrecken und gemeinsam die Mantelfläche des Formrohrs bilden. Durch die Formrohre ist der Außenrahmen des Gärschranks besonders stabil. Der Winkel bezeichnet dabei jeweils denjenigen spitzen Winkel, der zwischen einer gedachten lotrechten Geraden durch das jeweilige Formrohr und der in Richtung des Innenraums geneigten Seitenfläche bzw. auch mehreren in Richtung des Innenraums geneigten Seitenflächen des Formrohrs eingeschlossen ist. Diese Neigung der Seitenflächen stellt sicher, dass sich vorteilhafterweise auf den Formrohren des Außenrahmens kein Material mehr anlagert bzw. leicht bei der Reinigung entfernen oder abwaschen lässt.

Als Transportebenen werden in diesem Zusammenhang diejenigen im Wesentlichen horizontal oder vertikal verlaufenden Ebenen verstanden, entlang denen Teiglinge von der Fördervorrichtung annähernd horizontal oder vertikal durch den Gärschrank transportiert werden. Alle Rahmenprofile, die sich - bei horizontal ausgerichteten Transportebenen - unterhalb der in Schwerkraftrichtung gesehen zu oberst angeordneten Transportebene befinden oder - bei vertikal angeordneten Transportebenen - unterhalb desjenigen Punktes der Transportebenen befinden, der in Schwerkraftrichtung gesehen zu oberst angeordnet ist, sind bei einem erfindungsgemäßen Gärschrank in Richtung des Innenraums geneigt.

Bei Gärschränken, die eine Anzahl von annähernd vertikalen, parallel zueinander verlaufenden Transportebenen aufweisen, ist als oberster Punkt die Umlenkung am höchsten Punkt der Transportebenen zu verstehen. Bei Gärschranken, die eine Anzahl von annähernd horizontalen, parallel zueinander verlaufenden Transportebenen aufweisen, liegt der oberste Punkt jeweils in der obersten Transportebene, d.h. derjenigen Transportebene, die in Schwerkraftrichtung zuoberst angeordnet ist, sodass als oberster Punkt jeweils die oberste Transportebene, die in Schwerkraftrichtung zuoberst angeordnet ist, zu verstehen ist.

Ein Außenrahmen, in dem vorteilhafterweise besonders wenige Nischen und Öffnungen, vorhanden sind, in denen sich möglicherweise Verunreinigungen und Ungeziefer ansammeln können, kann ausgebildet werden, wenn der Außenrahmen öffnungsfrei, insbesondere bohrungsfrei, ausgebildet ist.

Besonders vorteilhaft für die Reinigung des Gärschranks ist es, wenn die Formrohre derart ausgerichtet sind, dass zwischen der Schwerkraftrichtung und den in Richtung des Innenraums geneigten Seitenflächen der normal zur Schwerkraftrichtung verlaufenden Formrohre ein Winkel von kleiner als 45°, vorzugsweise kleiner als 30°, eingeschlossen ist, sodass Wasser und Verunreinigungen in den Innenraum des Gärschranks abfließen.

Konstruktiv besonders einfach kann der Außenrahmen für einen derartigen Gärschrank hergestellt werden, wenn zwischen der Schwerkraftrichtung und den in Richtung des Innenraums geneigten Seitenflächen der normal zur Schwerkraftrichtung verlaufenden Formrohre ein Winkel von 45° eingeschlossen ist. Besonders vorteilhaft für eine einfache Verbindung der Formrohre miteinander kann in diesem Zusammenhang sein, dass an den von jeweils drei Formrohren gebildeten Ecken des Gärschranks bei der normal zur Schwerkraftrichtung verlaufenden Formrohren jeweils eine der Flächendiagonalen der Querschnittsfläche parallel und die jeweils andere Flächendiagonale normal zur Schwerkraftrichtung ausgerichtet ist und die Längskanten der Formrohre jeweils einen rechten Winkel miteinander einschließen. Die Querschnittsfläche bezeichnet dabei diejenige Fläche, die sich ergibt, wenn die von den vier Seitenflächen gemeinsam aufgespannte Mantelfläche im Querschnitt betrachtet wird. Die beiden Flächendiagonalen verbinden dabei jeweils einander gegenüberliegende Ecken dieser aufgespannten Querschnittsfläche.

Um einen Gärschrank herzustellen, in dessen Außenrahmen keine Nischen für beispielsweise Bakterien oder Verunreinigungen bestehen, kann vorgesehen sein, dass die Rahmenprofile, gegebenenfalls die Formrohre, des Außenrahmens permanent stoffschlüssig, insbesondere durch Verschweißen, verbunden sind.

Um einen besonders flexiblen Einsatz eines Gärschranks z.B. für verschiedenste Teigarten und Teigkonsistenzen zu gewährleisten, kann vorgesehen sein, dass die Fördervorrichtung als Platten- oder Band-Fördervorrichtung oder als Fördervorrichtung, an der Gärgutträger, insbesondere Teigtassen, zur Aufnahme von Teiglingen angeordnet sind, ausgebildet ist. Typische Gärgutträger, die in Gärschränken Verwendung finden sind beispielsweise Teigtassen.

Das unerwünschte Austreten von Flüssigkeit aus dem Gärschrank bzw. unerwünschtes Eindringen von Feuchtigkeit, Verschmutzungen oder Ungeziefer in den Gärschrank kann vorteilhafterweise vermieden werden, wenn die Abdeckpaneele am Außenrahmen derart angeordnet sind, dass eine geschlossene Außenverkleidung um den Innenraum des Gärschranks mit einem Boden, einer dem Boden gegenüberliegenden Decke und Seitenwänden gebildet ist, sodass der Innenraum des Gärschranks flüssigkeitsdicht verschlossen ist.

Eine besonders einfache Herstellung eines erfindungsgemäßen Gärschranks bei gleichzeitiger Sicherstellung einer effizienten Reinigung kann gewährleistet werden, wenn die normal zur Schwerkraftrichtung verlaufenden Formrohre des Außenrahmens derart ausgerichtet sind, dass eine Längskante in Schwerkraftrichtung weiter vom Boden entfernt ist, als die übrigen drei Längskanten des jeweiligen Formrohrs. Dies bedeutet beispielsweise, dass Formrohre mit einem quadratischen Querschnitt derart in Richtung des Innenraums geneigt bzw. rotiert sind, dass beispielsweise eine Flächendiagonale des jeweiligen Formrohrs normal und eine Flächendiagonale in Schwerkraftrichtung verlaufen, sodass zwei Längskanten in gleicher Entfernung in Schwerkraftrichtung vom Boden der Außenverkleidung verlaufen. Die beiden anderen Längskanten, die im Querschnitt durch die parallel zur Schwerkraftrichtung verlaufende Flächendiagonale verbunden sind, verlaufen in diesem Fall jeweils eine in einem geringeren und eine in einem größeren Abstand vom Boden der Außenverkleidung gemessen in Schwerkraftrichtung.

Diese Ausrichtung der Flächendiagonalen der Formrohre ist jedoch keinesfalls zwingend und der vorteilhafte Effekt wird auch erzielt, wenn die Flächendiagonalen in einer anderen Ausrichtung zur Schwerkraftrichtung orientiert sind, bei der eine der vier Längskanten weiter vom Boden der Außenverkleidung entfernt ist als die übrigen drei Längskanten des jeweiligen Formrohrs.

Weiters vorteilhaft für eine effiziente Reinigung eines erfindungsgemäßen Gärschranks bei gleichzeitig einfacher Ausbildung des Außenrahmens, kann sein, dass die parallel zur Schwerkraftrichtung verlaufenden Formrohre derart ausgerichtet sind, dass jeweils zwei ihrer Seitenflächen dem Innenraum des Gärschranks zugewandt sind.

Für die Ausbildung einer beispielsweise flüssigkeitsdicht verschlossenen Außenverkleidung, die einfach vom Außenrahmen für eine gründliche Reinigung abzunehmen ist und gleichzeitig keine Nischen bietet, in denen Verunreinigungen haften bleiben können, kann vorgesehen sein, dass die, die Decke bildenden, Decken-Abdeckpaneele und die Formrohre des Außenrahmens reversibel formschlüssig verbunden sind. Besonders vorteilhaft kann in diesem Zusammenhang sein, dass die Decken-Abdeckpaneele an zumindest eine, vorzugsweise zwei, Seitenflächen jedes der Formrohre angepasste Auflageflächen aufweisen, da die Decken-Abdeckpaneele in diesem Fall ohne Nischen zu bilden auf dem Außenrahmen bzw. den Formrohren aufliegen und außerdem werkzeuglos entfernbar sind. Eine Außenverkleidung, die mit einer besonders geringen Anzahl an Decken-Abdeckpaneelen auskommt, wird bereitgestellt, wenn die Decken-Abdeckpaneele jeweils auf zumindest zwei Formrohren des Außenrahmens aufliegen.

Eine besonders einfach zu reinigende Außenverkleidung, die gleichzeitig einfach abzunehmen ist, kann bei einem erfindungsgemäßen Gärschrank bereitgestellt werden, wenn die den Boden bildenden Boden-Abdeckpaneele und die Formrohre des Außenrahmens reversibel formschlüssig verbunden sind und die Boden-Abdeckpaneele jeweils auf zumindest zwei Formrohren des Außenrahmens aufliegen oder in Führungsschienen in oder unter den Außenrahmen einschiebbar sind. Besonders vorteilhaft kann in diesem Zusammenhang sein, wenn die Boden-Abdeckpaneele zumindest eine in ihrer Neigung an eine Seitenfläche der Formrohre angepasste Außenwand und vorzugsweise zumindest eine entlang der Außenwand verlaufende Griffleiste aufweisen, da derartige Boden-Abdeckpaneele auch beispielsweise als Mehlauffanglade dienen können, die einfach beispielsweise unter den Außenrahmen geschoben und wieder entfernt und gereinigt werden kann.

Um einen einfachen Zugang ins Innere des Gärschranks für beispielsweise Wartungs- oder Reinigungsarbeiten zu gewährleisten, kann vorgesehen sein, dass die die Seitenwände bildenden Seiten-Abdeckpaneele aufschwenkbar an den Formrohren des Außenrahmens angeordnet sind. Um einen einfachen Ausbau derartiger Seiten-Abdeckpaneele beispielsweise für Reinigungsarbeiten zu gewährleisten, kann vorgesehen sein, dass die Seiten-Abdeckpaneele mittels in Bohrungen in den vorzugsweise normal zur Schwerkraftrichtung verlaufenden Formrohren einsteckbaren oder auf an den Formrohren angeschweißten Aufhängungen befestigten Scharnieren am Außenrahmen angeordnet sind.

Um zu verhindern, dass die Seiten-Abdeckpaneele unbeabsichtigt geöffnet werden, kann vorgesehen sein, dass die Seiten-Abdeckpaneele aus einer Schließstellung, in der die Seiten-Abdeckpaneele an zumindest zwei Formrohren des Außenrahmens aufliegen, verschwenkbar sind und dass an den Seiten-Abdeckpaneelen jeweils zumindest ein Schalter angeordnet ist, der dazu ausgebildet ist, ein Verschwenken der Seiten-Abdeckpaneele aus der Schließstellung zu detektieren. Dies bedeutet, dass der Schalter feststellt, wenn ein Seiten-Abdeckpaneel geöffnet wird, wobei zusätzlich vorgesehen sein kann, dass in diesem Fall beispielsweise ein Warnsignal abgegeben wird, sodass die Bedienperson darauf aufmerksam gemacht wird, dass ein Seiten-Abdeckpaneel des Gärschranks geöffnet ist.

Um sicherzustellen, dass auch Halterungen für die Antriebselemente der im Gärschrank verlaufenden Fördervorrichtung wie beispielsweise Ketten, Kettenräder oder Wellen einfach zu reinigen sind, kann vorgesehen sein, dass an den Rahmenprofilen, gegebenenfalls an den Formrohren, des Außenrahmens Halterungen für Antriebselemente der Fördervorrichtung reversibel formschlüssig angeordnet sind, insbesondere verschraubt sind. Diese Ausgestaltung der Halterungen stellt vorteilhafterweise sicher, dass diese von allen Seiten für Reinigungsarbeiten zugänglich sind.

Die Erfindung betrifft weiters einen Garschrank mit einer Fördervorrichtung, die ein Riemen- oder Kettengetriebe mit einem Riemen- oder Kettensystem und einer Spannvorrichtung zum selbstständigen Spannen des Riemen- oder Kettensystems umfasst. Erfindungsgemäß ist dabei für eine einfache Reinigung des Riemen- oder Kettensystems vorgesehen, dass die Spannvorrichtung zumindest einen Spannzylinder mit einem darin beweglichen Kolben umfasst. Die Spannvorrichtung ist dabei derart zwischen dem Außenrahmen und einer Riemenscheibe oder einem Kettenrad des Riemen- oder Kettensystems angeordnet, dass bei Kräfteveränderung im Riemen- oder Kettensystem die Riemenscheibe oder das Kettenrad relativ zum Außenrahmen verstellbar ist. Eine derartige Ausgestaltung der Spannvorrichtung stellt vorteilhafterweise sicher, dass das Riemen- oder Kettensystem zur Reinigung des Gärschranks einfach entfernt und anschließend mühelos wieder eingebaut werden kann, ohne dass ein aufwendiges händisches Spannen des Riemen- oder Kettensystems erforderlich ist.

Eine besonders einfache Reinigung der Spannvorrichtung kann erzielt werden, wenn die Spannvorrichtung am Außenrahmen, insbesondere an den am Außenrahmen angeordneten Halterungen für die Antriebselemente der Fördervorrichtung, angeordnet ist.

Eine einfache Reinigung des Innenraums des Gärschranks von Verunreinigungen wie beispielsweise anhaftendem Mehl bzw. eine effiziente Selbstreinigung des Gärschranks kann gewährleistet werden, wenn eine Reinigungsvorrichtung zur Reinigung des Innenraums vorgesehen ist, die zumindest eine, insbesondere am Außenrahmen angeordnete, Wasserabgabevorrichtung und/oder zumindest eine Ablauföffnung umfasst, wobei die Ablauföffnung in den den Boden der Außenverkleidung bildenden Boden-Abdeckpaneelen ausgebildet ist.. So können beispielsweise Wasserdüsen am Außenrahmen oder den Decken-Abdeckpaneelen angeordnet sein, die für die Reinigungsarbeiten am Gärschrank Wasser abgeben, sodass Verunreinigungen abgespült werden und durch die zumindest eine Ablauföffnung im Boden abfließen.

Eine effiziente Reinigung der Gärgutträger kann erzielt werden, wenn eine Reinigungsstation zur Reinigung der Gärgutträger vorgesehen ist, die im Innenraum des Gärschranks oder in einem an den Innenraum angeschlossenen Zusatzgehäuse angeordnet ist und zumindest eine Absaugvorrichtung und/oder Bürsten und/oder zumindest eine Wasserabgabevorrichtung umfasst. Um einen automatisierten Transport der Gärgutträger in die Reinigungsstation zu gewährleisten und somit ein manuelles Ausbauen zu vermeiden, kann dabei vorgesehen sein, dass die Fördervorrichtung dazu ausgebildet ist, die Gärgutträger durch die Reinigungsstation zu transportieren.

Um zusätzlich zur Reinigung der Gärgutträger auch eine Trocknungs- und Sterilisationsmöglichkeit für die Gärgutträger bereitzustellen, kann vorgesehen sein, dass die Reinigungsstation eine Trocknungsvorrichtung zur Trocknung der Gärgutträger, insbesondere mittels Infrarotstrahlung, und/oder eine Entkeimungsvorrichtung zur Desinfektion der Gärgutträger umfasst. Eine besonders effiziente Desinfektion der Gärgutträger kann erzielt werden, wenn die Entkeimungsvorrichtung zumindest eine Vorrichtung zum Auftragen von Wasserstoffperoxid auf die Gärgutträger und/oder zumindest ein Beleuchtungsmittel zur Bestrahlung der Teigtrassen mit ultraviolettem Licht umfasst.

Ein weiterer Aspekt der Erdfindung sieht vor, ein Verfahren zum Reinigen eines Gärschranks bereit zu stellen. Dabei ist erfindungsgemäß vorgesehen, dass zumindest eine Profilfläche der in einem Winkel zur Schwerkraftrichtung verlaufenden Rahmenprofile des Außenrahmens in Richtung des Innenraums geneigt ist, und dass der auf zumindest einer Profilfläche abgelagerte Schmutz mit der Reinigungsvorrichtung abgewaschen wird, wobei sich auf der Profilfläche befindliches Wasser und/oder Reinigungsmittel und/oder Schmutz durch die Neigung der Profilfläche, insbesondere vollständig, in den Innenraum abläuft.

Weitere Vorteile und Ausgestaltungen der Erfindung ergeben sich aus der Beschreibung und den beiliegenden Zeichnungen.

Die Erfindung ist im Folgenden anhand von besonders vorteilhaften, aber nicht einschränkend zu verstehenden Ausführungsbeispielen in den Zeichnungen schematisch dargestellt und wird unter Bezugnahme auf die Zeichnungen beispielhaft beschrieben.

Im Folgenden zeigen:
Fig. 1 eine Frontalansicht eines Ausführungsbeispiels eines erfindungsgemäßen Gärschranks,
Fig. 2 eine Draufsicht auf den erfindungsgemäßen Gärschrank,
Fig. 3 einen Querschnitt durch das Ausführungsbeispiel,
Fig. 4 eine Seitenansicht des Ausführungsbeispiels,
Fig. 5 eine Schrägansicht des Ausführungsbeispiels,
Fig. 6 eine Detailansicht einer Schnittdarstellung im Bereich der Decke,
Fig. 7 ein Scharnier zur Befestigung der Seiten-Abdeckpaneele,
Fig. 7a zeigt ein zweites Ausführungsbeispiel eines Scharniers zur Befestigung der Seiten-Abdeckpaneele,
Fig. 8 einen Längsschnitt durch das Ausführungsbeispiel,
Fig. 9 eine Schrägansicht des Außenrahmens,
Fig. 10 ein Boden-Abdeckpaneel im Detail,
Fig. 11 eine Detailansicht eines Decken-Abdeckpaneels,
Fig. 12 eine Detailansicht der Spannvorrichtung im eingebauten Zustand,
Fig. 13 eine Detailansicht eines Seiten-Abdeckpaneels,
Fig. 14 eine Detailansicht einer Ecke des ersten Ausführungsbeispiels.

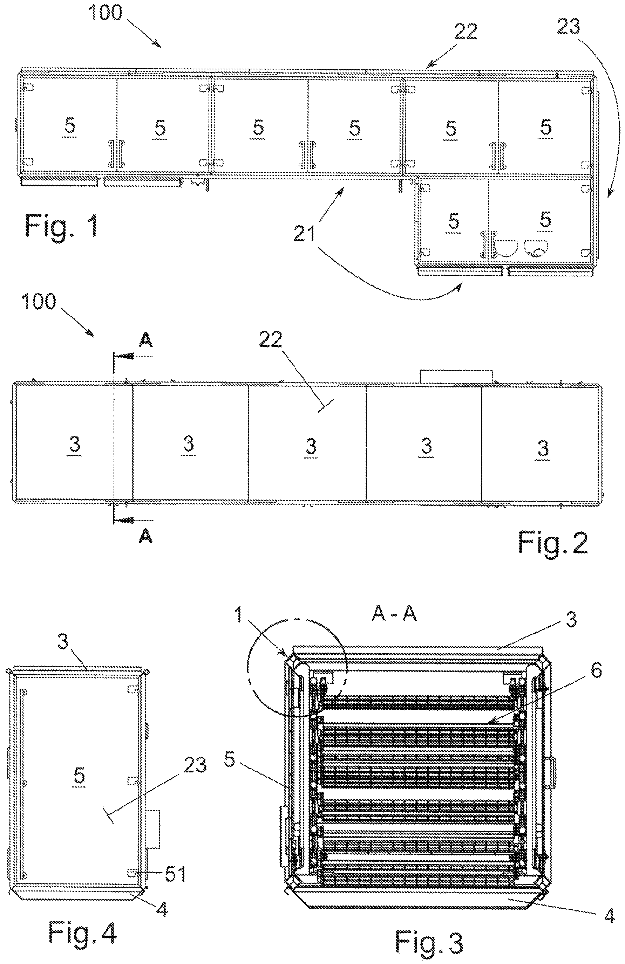

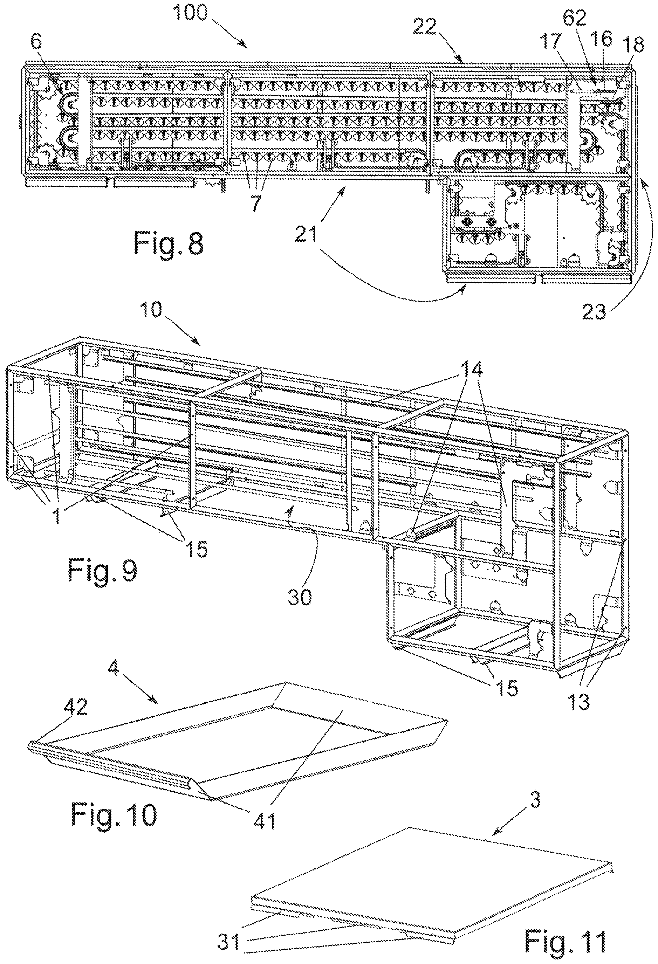

Die Fig. 1, 2, 4 und 5 zeigen jeweils ein Ausführungsbeispiel eines erfindungsgemäßen Gärschranks 100 zum Gären von Teig, der eine rechteckige Grundfläche und eine ebenfalls rechteckige Querschnittsfläche aufweist. Der Gärschrank 100 umfasst einen Außenrahmen 10, der einen Innenraum 30 des Gärschranks umschließt (Fig. 9) und der in Schwerkraftrichtung bzw. in einem Winkel zur Schwerkraftrichtung verlaufende Rahmenprofile aufweist, bei denen es sich im Ausführungsbeispiel um Formrohre 1 handelt, wie dies in Fig. 9 im Detail dargestellt ist. Der Außenrahmen 10 setzt sich im gezeigten Ausführungsbeispiel aus zwei einzelnen Bereichen bzw. Modulen mit jeweils rechteckiger Querschnitts- und Grundfläche zusammen. Durch den Aufbau aus Formrohren 1 kann der Außenrahmen 10 einfach um weitere derartige Module erweitert werden, in denen verschiedene Vorrichtungen für weitere Teigverarbeitungsschritte untergebracht sein können.

Weiters umfasst der Gärschrank 100 eine Fördervorrichtung 6, an der im gezeigten Ausführungsbeispiel Gärgutträger 7 zur Aufnahme von Teigportionen, die als Teigtassen ausgebildet sind, angeordnet sind. Alternativ dazu kann die Fördervorrichtung 6 auch als Platten- oder Bandfördervorrichtung ausgebildet sein.

Die Fördervorrichtung 6 spannt im gezeigten Ausführungsbeispiel mehrere horizontal ausgerichtete Transportebenen auf, entlang denen Teiglinge durch den Gärschrank 100 transportiert werden. Alternativ dazu kann die Fördervorrichtung 6 eines erfindungsgemäßen Gärschranks 100 auch eine Anzahl von vertikal ausgerichteten Transportebenen aufspannen.

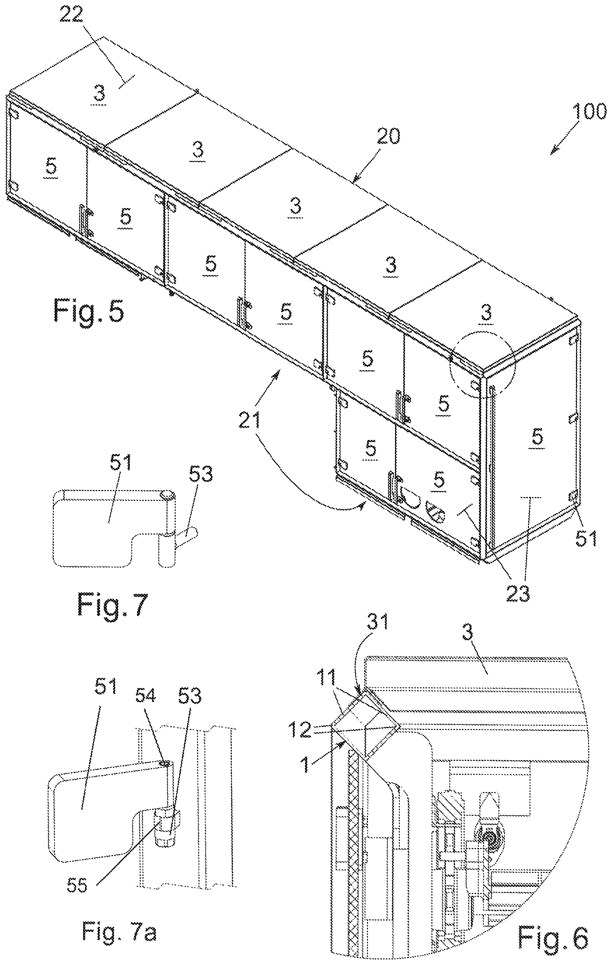

Wie insbesondere in Fig. 1 und Fig. 5 ersichtlich ist, umfasst der Außenrahmen 10 im gezeigten Ausführungsbeispiel zwei Bereiche, sodass vorteilhafterweise Teigportionen in den Gärgutträger 7 nicht nur von der Fördervorrichtung 6 zum Gären bzw. Gehenlassen des Teigs durch den Innenraum 30 transportiert werden, was in Fig. 8 im linken bzw. oberen Bereich des Gärschranks 100 dargestellt ist. Durch eine Erweiterung des Innenraums 30 mit einem zweiten Außenrahmen-Modul werden im gezeigten Ausführungsbeispiel auch weitere Teigverarbeitungsschritte wie beispielsweise ein Langrollen im Innenraum 30 des Gärschranks 100 durchgeführt.

Der erfindungsgemäße Gärschrank 100 umfasst weiters eine Anzahl an am Außenrahmen 10 angeordneten Abdeckpaneelen 3, 4, 5, die gemeinsam eine geschlossene Außenverkleidung 20 bilden.

Der Gärschranks 100 weist weiters eine im Innenraum 30 verlaufende Fördervorrichtung 6 auf, an der Gärgutträger 7 zur Aufnahme von Teigportionen angeordnet sind, die in Fig. 8 bzw. Fig. 3 näher ersichtlich sind. Die Fördervorrichtung 6 ist dazu ausgebildet, die Gärgutträger 7 durch den Gärschrank 100 zu transportieren.

Optional kann ein erfindungsgemäßer Gärschrank 100 auch Mittel zur Herstellung einer gleichbleibenden Atmosphäre im Innenraum 30 des Gärschranks 100 mit kontrollierten Luftfeuchte- und Temperaturbedingungen, die das Gären Teigs fördern, umfassen.

Die Rahmenprofile des Außenrahmens 10 des erfindungsgemäßen Gärschranks verlaufen dabei so, dass zumindest eine Profilfläche jedes der in einem Winkel zur Schwerkraftrichtung ausgerichteten Rahmenprofile derart in Richtung des Innenraums 30 geneigt ist, dass im Gärschrank 100 befindliche Verschmutzungen und Wasser in den Innenraum 30 abfließen. Wie in Fig. 9 ersichtlich ist, sind die Rahmenprofile im gezeigten Ausführungsbeispiel als Formrohre 1 ausgebildet und weisen einen quadratischen Querschnitt auf sowie vier Längskanten 12 und vier Seitenflächen 11, was in der Detailansicht in Fig. 6 näher dargestellt ist.

Im gezeigten Ausführungsbeispiel ist der Außenrahmen 10 aus normal und parallel zur Schwerkraftrichtung verlaufenden Formrohren gebildet. Die normal zur Schwerkraftrichtung verlaufenden Formrohre sind in einem Winkel von 45° in Richtung des Innenraums 30 rotiert bzw. geneigt. Wie in Fig. 6 gezeigt ist, bedeutet dies, dass die beiden in Richtung des Innenraums 30 geneigten Seitenflächen 11 eines jeden normal zur Schwerkraftrichtung verlaufenden Formrohrs 1 einen spitzen Winkel von 45° mit der Schwerkraftrichtung einschließen.

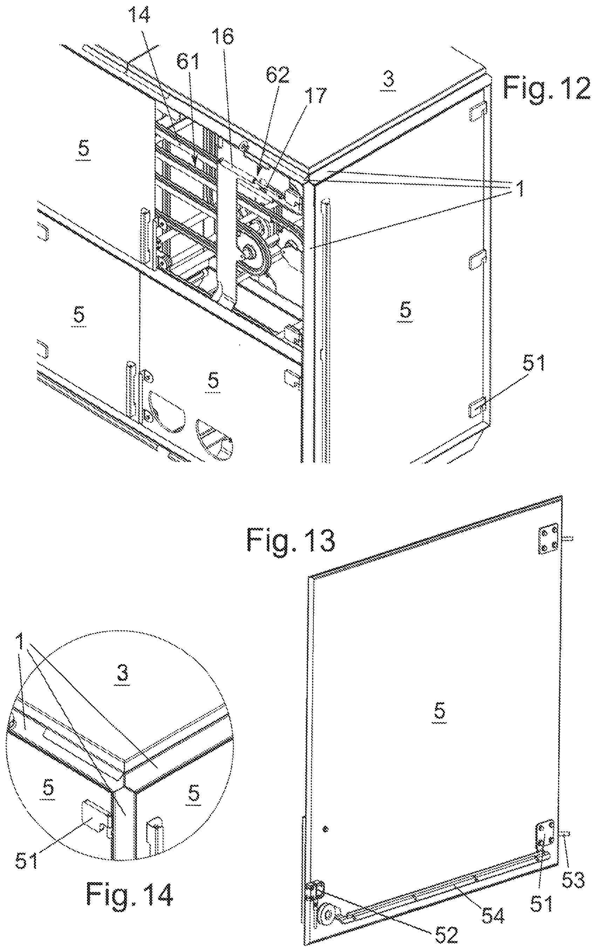

In Fig. 6 ist weiters ersichtlich, dass im gezeigten Ausführungsbeispiel die Flächendiagonalen der Querschnittsfläche der normal zur Schwerkraftrichtung verlaufenden Formrohre 1 parallel bzw. normal zur Schwerkraftrichtung verlaufen, sodass eine der Längskanten 12 weiter vom Boden entfernt ist als die restlichen drei Längskanten 12. Fig. 14 zeigt eine Detailansicht einer Ecke des Außenrahmens 10 des erfindungsgemäßen Gärschranks 100, an der drei Formrohre 1 derart miteinander verbunden sind, dass zwei Seitenflächen 11 des parallel zur Schwerkraftrichtung verlaufenden Formrohrs 1 in den Innenraum 30 des Gärschranks 100 orientiert sind und die Flächendiagonalen parallel bzw. normal zur Schwerkraftrichtung stehen.

Alternativ dazu können diejenigen Rahmenprofile bzw. Formrohre 1 des Außenrahmens 10, die in einem Winkel zur Schwerkraftrichtung verlaufen und oberhalb derjenigen Transportebene oder desjenigen Punktes der Transportebenen angeordnet sind, die bzw. der in Schwerkraftrichtung am obersten angeordnet ist, z.B. auch waagrecht verlaufen, und nicht in Richtung des Innenraums 30 geneigt sein.

Diese Ausgestaltung des Außenrahmens 10 stellt vorteilhafterweise sicher, dass beispielsweise Mehl, das, während die Gärgutträger 7 mittels der Fördervorrichtung 6 durch den Gärschrank 100 transportiert werden, von den Gärgutträger 7 abrieselt, nicht an den Formrohren 1 haften bleibt, sondern direkt in den Innenraum 30 des Gärschranks 100 abgleitet bzw. bei der Reinigung des Gärschranks 100 mit Wasser einfach von den Formrohren 1 abgespült werden kann.

Um zu vermeiden, dass sich in etwaigen Verbindungsstellen zwischen den Formrohren 1 des Außenrahmens 10 Nischen bilden, in denen sich beispielsweise Verunreinigungen wie Mehl ansammeln können oder eventuell Ungeziefer einnistet, sind die Formrohre 1 im Ausführungsbeispiel miteinander verschweißt. Alternativ dazu können die Formrohre auch auf andere Weise stoffschlüssig verbunden sein.

Die in den Fig. 1, 2, 4 und 5 dargestellte Außenverkleidung 20 des Gärschranks 100 ist durch Anordnen einer Anzahl von Abdeckpaneelen 3, 4, 5 am Außenrahmen 10 gebildet, sodass die Außenverkleidung 20 um den Innenraum 30 des Gärschranks 100 geschlossen ausgebildet ist. Die Außenverkleidung 20 umfasst einen Boden 21, Seitenwände 23 und eine dem Boden 21 gegenüberliegende Decke 22. Die Außenverkleidung 20 verschließt den Innenraum 30 des Gärschranks 100 flüssigkeitsdicht, sodass weder Flüssigkeit aus dem Innenraum 30 austreten kann, noch unerwünschterweise Feuchtigkeit in diesen eindringen kann.

Der Boden 21 der Außenverkleidung 20 wird im gezeigten Ausführungsbeispiel von quadratischen bzw. rechteckigen Boden-Abdeckpaneelen 4 aus Blech gebildet, die mit den Formrohren 1 des Außenrahmens 10 reversibel formschlüssig verbunden sind. Im gezeigten Ausführungsbeispiel liegen die Boden-Abdeckpaneele 4 jeweils auf zumindest zwei Formrohren 1 des Außenrahmens 10 auf oder sind in Führungsschienen 15 unter dem Außenrahmen 10 einschiebbar.

Bei den einschiebbaren Boden-Abdeckpaneelen 4 handelt es sich im gezeigten Ausführungsbeispiel um als Mehlauffangladen ausgebildete Boden-Abdeckpaneele 4, die in ihre Neigung an zwei Seitenflächen 11 der Formrohre 1 angepasste Außenwände 41 und eine entlang einer Außenwand 41 verlaufende Griffleiste 42 aufweisen. Diese Boden-Abdeckpaneele 4 sind vorteilhafterweise derart ausgestaltet, dass die in den Führungsschienen 15 geführten Seitenwände senkrecht verlaufen, während die an den Rahmenprofilen anliegenden Seitenwände 41 abgeschrägt sind, sodass vom Außenrahmen 10 abgleitende Verunreinigungen einfach in das Boden-Abdeckpaneel 4 gelangen und dort gesammelt werden. Dadurch dass diese Boden-Abdeckpaneele 4 unter den Außenrahmen 10 einschiebbar sind, können diese einfach im Betrieb des Gärschranks 100 oder während eines Wartungs- oder Reinigungsvorgangs ausgezogen und darin aufgefangenen Verunreinigungen entfernt werden.

Die Decke 22 der Außenverkleidung 20 ist im gezeigten Ausführungsbeispiel aus quadratischen Decken-Abdeckpaneelen 3 aus Blech gebildet, die mit den Formrohren 1 des Außenrahmens 10 reversibel formschlüssig verbunden sind. Alternativ können die Decken-Abdeckpaneele 3 beispielsweise auch rechteckig ausgebildet sein. Dieser Formschluss wird erzielt, indem die Decken-Abdeckpaneele 3 Auflageflächen 31 aufweisen, die an die beiden Seitenflächen 11 des jeweiligen Formrohrs 1 angepasst sind, auf dem das Decken-Abdeckpaneel 3 aufliegt. Die formschlüssige Verbindung zwischen einem Decken-Abdeckpaneel 3 und einem Formrohr 1 ist im Detail in Fig. 6 dargestellt und stellt sicher, dass die Decken-Abdeckpaneele 3 abgenommen werden können, ohne dass hierfür Werkzeug notwendig ist. Alternativ dazu können die Decken-Abdeckpaneele 3 reversibel formschlüssig beispielsweise mittels Einstecken von Haltestiften in die Formrohre 1 am Außenrahmen 10 angebracht sein.

Die Ausgestaltung des Decken-Abdeckpaneels 3 mit Auflageflächen 31 stellt sicher, dass durch die Neigung des Formrohrs 1 in Richtung des Innenraums 30 keine Nischen entstehen, in denen sich Verunreinigungen anlagern, wie dies bei gerade verlaufenden Decken-Abdeckpaneelen 3 der Fall wäre. Zusätzlich sind durch die formschlüssige Verbindung die Decken-Abdeckpaneele 3 ausreichend fest mit dem Außenrahmen 10 verbunden, sodass diese nicht unerwünschterweise von den Formrohren 1 abrutschen können. Gleichzeitig ist sichergestellt, dass diese werkzeuglos entfernbar sind und einfach von den Formrohren 1 abgenommen werden können, um beispielsweise Reinigungs- oder Wartungsarbeiten durchzuführen oder die Decken-Abdeckpaneele 3 auszutauschen.

Die Seitenwände 23 der Außenverkleidung 20 werden im gezeigten Ausführungsbeispiel von quadratischen bzw. rechteckigen Seiten-Abdeckpaneelen 5 aus Blech gebildet, die aufschwenkbar an den Formrohren 1 des Außenrahmens 10 angeordnet sind. Alternativ können die Seiten-Abdeckpaneele 5 beispielsweise auch aus Plexiglas ausgebildet sein, um den Ablauf der Verarbeitungsschritte im Inneren 30 des Gärschranks 100 verfolgen zu können, wenn die Seitenwände 23 geschlossen sind.

Im Außenrahmen 10 sind in den parallel zur Schwerkraftrichtung verlaufenden Formrohren 1 Bohrungen 13 (Fig. 9) angeordnet, die zur Fixierung der Seiten-Abdeckpaneele 5 am Außenrahmen 10 dienen. Zur schwenkbaren Fixierung der Seiten-Abdeckpaneele 5 sind in die Bohrungen 13 einsteckbare Scharniere 51 an den Seiten-Abdeckpaneelen 5 angeordnet. Die Scharniere 51 weisen dazu einen Fortsatz 53 auf, der jeweils in eine Bohrung 13 in einem Formrohr 1 des Außenrahmens 10 einsteckbar ist. Dadurch ist gleichzeitig eine ausreichende Fixierung der Seiten-Abdeckpaneele 5 am Außenrahmen 10 sowie ein leichtes Verschwenken der Seiten-Abdeckpaneele 5 gewährleistet, während gleichzeitig durch einfaches Herausziehen der Scharniere 51 aus dem Außenrahmen 10 ein einfaches Austauschen der Seiten-Abdeckpaneele 5 möglich ist. Die Scharniere 51 stellen weiters sicher, dass die Seiten-Abdeckpaneele 5 weit geöffnet werden können, um einen einfachen Zugang zum Innenraum 30 des Gärschranks 100 zu ermöglichen.

Alternativ dazu können, wie in Fig. 7a dargestellt, die Seiten-Abdeckpaneele 5 mittels Scharnieren 51 am Außenrahmen 10 angeordnet sein und derart Türen bilden, wobei die Scharniere 51 in einem Stift 54 aufgehängt sind. Der Stift 54 ist in eine Aufhängung 55, die an dem Außenrahmen 10 angeschweißt ist, eingeführt und mittels Muttern gegen ein Herausfallen gesichert. Der Stift 54 ist zweiteilig ausgebildet, wobei der erste Teil in der Aufhängung 55 angeordnet ist und auf dem zweiten Teil das Scharnier 51 steckt. Der zweite Teil ist zu dem ersten Teil exzentrisch angeordnet, sodass der Abstand des Scharniers 51 bzw. das jeweilige an dem Scharnier 51 befestigte Seiten-Abdeckpanel 5 durch verdrehen des Stiftes verändert werden kann.

Dies bietet den zusätzlichen Vorteil, dass der Außenrahmen 10 in diesem Fall völlig bohrungsfrei ausgestaltet ist und somit weniger Nischen, in denen sich möglicherweise Verunreinigen ablagern können, vorhanden sind.

Die Seiten-Abdeckpaneele 5 liegen im gezeigten Ausführungsbeispiel an zumindest zwei Formrohren 1 des Außenrahmens 10 auf und weisen einen Schalter 52 auf, der dazu ausgebildet ist, zu überprüfen, ob ein Kontakt zwischen dem Seiten-Abdeckpaneel 5 und dem Formrohr 1 vorherrscht und derart ein Verschwenken der Seiten-Abdeckpaneele aus einer Schließstellung, in der diese an zumindest zwei Formrohren 1 des Außenrahmens 10 aufliegen, zu detektieren. Wird vom Schalter 52 ein Verschwenken der Seiten-Abdeckpaneele 5 detektiert, so ist es beispielsweise möglich, dass die Detektionsinformation an eine Steuerzentrale, von der aus der Gärschrank 100 bedient wird, übermittelt wird oder beispielsweise ein optisches oder akustisches Warnsignal an den Bediener des Gärschranks 100 abgegeben wird. Somit ist sichergestellt, dass ein unerwünschtes Öffnen der Außenverkleidung 20 des Gärschranks 100 rasch festgestellt und behoben wird.

Wie in Fig. 8 ersichtlich ist, tritt die Fördervorrichtung 6 im Bereich des Bodens 21 der Außenverkleidung 20 in den Innenraum 30 des Gärschranks 100 ein und ist mit einem Kettengetriebe mit einem Kettensystem 61 angetrieben. Entlang der Längserstreckung des Gärschranks 100 ist das Kettensystem 61 dabei mehrere Male über Kettenräder 18 längs im Innenraum 30 hin und her geführt, sodass die an der Fördervorrichtung 6 angeordneten Gärgutträger 7 über einen vorgegebenen Zeitraum im Innenraum 30 des Gärschranks 100 verbleiben bzw. darin transportiert werden. Die Gärgutträger 7 sind dabei normal zur Transportrichtung der Fördervorrichtung 6 angeordnet, um mehrere Teigportionen auf einer Teigtasse 7 unterbringen zu können.

Um ein ideales Gären des Teigs zu gewährleisten, können bei einem erfindungsgemäßen Gärschrank Mittel zur Herstellung konstanter Luftfeuchte- und Temperaturbedingungen wie beispielsweise eine Heizvorrichtung oder Klimaanlage und Wassersprüheinrichtungen im Innenraum 30 vorgesehen sein.

Im gezeigten Ausführungsbeispiel sind Halterungen 14 für die Antriebselemente der Fördervorrichtung 6 an den Formrohren 1 des Außenrahmens 10 reversibel formschlüssig angeordnet. Die Halterungen 14 sind im Ausführungsbeispiel mit dem Außenrahmen 10 derart verschraubt, dass sie von allen Seiten zugänglich sind und gereinigt werden können.

Um auch das Kettensystem 61 der Fördervorrichtung 6 einfach ausbauen und reinigen zu können, umfasst die Fördervorrichtung 6 im gezeigten Ausführungsbeispiel eine Spannvorrichtung 62, die das Kettensystem 61 selbstständig spannt. Ein Ausführungsbeispiel einer erfindungsgemäßen Spannvorrichtung 62 ist in Fig. 8 und Fig. 12 im Detail dargestellt und umfasst einen Spannzylinder 16 mit einem darin beweglichen Kolben 17. Der Kolben 17 ist derart zwischen dem Außenrahmen 10 und einem Kettenrad 18 des Kettensystems 61 angeordnet, dass bei Kräfteveränderung im Kettensystem 61 das Kettenrad 18 relativ zum Außenrahmen 10 verstellt wird. Dies kann beispielsweise erzielt werden, indem im Inneren des Zylinders 16 ein konstanter oder vorgebbarer Druck herrscht, der beispielsweise den Kolben 17 ausfahren lässt, wenn das Kettensystem 61 eine zu geringe Spannung aufweist und das Kettensystem 61 derart nachgespannt wird. Somit kann das Kettensystem 61 zur Reinigung des Gärschranks 100 einfach abgenommen und von Verschmutzungen befreit werden. Anschließend kann es mühelos wieder an den Halterungen 14 angeordnet werden, ohne es mühsam händisch nachspannen zu müssen. Die Spannvorrichtung 62 ist im gezeigten Ausführungsbeispiel an den Halterungen 14 für die Antriebselemente der Fördervorrichtung 6 am Außenrahmen 10 angeordnet.

Alternativ dazu kann die Spannvorrichtung 62 auch als Feder, beispielsweise als Gasdruckfeder, ausgebildet sein, in der ein konstanter oder vorgebbarer Gasdruck herrscht, sodass das Kettensystem 61 selbstständig von der Gasdruckfeder nachgespannt wird, wenn das Kettensystem 61 an Spannung verliert.

Bei allen Ausführungsformen eines erfindungsgemäßen Gärschranks 100 kann eine Reinigungsvorrichtung zur Reinigung des Innenraums 30 unter der Verwendung eines erfindungsgemäßen Verfahren vorgesehen sein, um ein aufwendiges händisches Reinigen des Innenraums 30 zu vermeiden. Die Reinigungsvorrichtung kann dabei am Außenrahmen 10 angeordnete Wasserabgabevorrichtungen und/oder zumindest eine Ablauföffnung im Boden 21 der Außenverkleidung 20 umfassen. Mit einer derartigen Reinigungsvorrichtung ist es beispielsweise möglich, während des Betriebs des Gärschranks oder in vorgegebenen Reinigungsintervallen Verunreinigungen, die eventuell im Innenraum 30 des Gärschranks 100 anhaften, abzulösen, sodass diese einfach durch die Ablauföffnung im Boden 21 abfließen. Dazu werden beispielsweise Wasserdüsen am Außenrahmen 10 des Gärschranks 100 aktiviert, die Wasser in den Innenraum 30 abgegeben, sodass Mehl- und Teigreste verflüssigt werden und von den geneigten Formrohren des Außenrahmens 10 bzw. der Außenverkleidung 20 oder der Fördervorrichtung 6 abfließen.

Da durch die Ausgestaltung des Außenrahmens 10 des Gärschranks 100 aus Rahmenprofilen bzw. Formrohren 1 dieser, wie zuvor beschrieben, besonders einfach erweiterbar ist, kann bei allen Ausführungsformen eines erfindungsgemäßen Gärschranks 100 eine Reinigungsstation zur Reinigung der Gärgutträger 7 vorgesehen sein. Die Reinigungsstation kann dabei im Inneren 30 des Gärschranks 100 untergebracht oder in einem an den Innenraum 30 angeschlossenen Zusatzgehäuse angeordnet sein. Um ein händisches Ausbauen der Gärgutträger 7 bzw. Abnehmen von der Fördervorrichtung 6 zu vermeiden, kann die Fördervorrichtung 6 durch die im Innenraum 30 angeordnete Reinigungsstation oder auch die in einem Zusatzgehäuse an den Gärschrank 100 angeschlossene Reinigungsstation verlaufen und dazu ausgebildet sein, die Gärgutträger 7 durch die Reinigungsstation zu transportieren. So kann vorgesehen sein, dass die Fördervorrichtung 6 in vorgegebenen Reinigungsintervallen oder auch im laufenden Betrieb die Teigtasse 7 durch die Reinigungsstation transportiert.

Die Reinigungsstation kann dabei zumindest eine Absaugvorrichtung und/oder Bürsten und/oder zumindest eine Wasserabgabevorrichtung umfassen. Somit besteht vorteilhafterweise die Möglichkeit, die Gärgutträger 7 mechanisch mittels Bürsten zu reinigen, und zusätzlich oder alternativ dazu eine Absaugung der Verschmutzungen vorzunehmen. Zusätzlich oder alternativ dazu kann eine Nassreinigung der Gärgutträger 7 mittels Wasserstrahl oder rotierenden Wasserdüsen vorgenommen werden.

Die Reinigungsstation kann zusätzlich dazu eine Trocknungsvorrichtung zur Trocknung der Gärgutträger 7 umfassen, wobei die Trocknung beispielsweise mittels Infrarotstrahlung oder auch Heißluftdüsen vorgenommen werden kann. Optional kann die Reinigungsstation weiters eine Entkeimungsvorrichtung zur Desinfektion der Gärgutträger 7 umfassen, die zumindest eine Vorrichtung zum Auftragen von Wasserstoffperoxid auf die Gärgutträger 7 und zusätzlich oder alternativ dazu ein Beleuchtungsmittel zur Bestrahlung der Gärgutträger 7 mit ultraviolettem Licht umfassen kann. Durch eine derartige Behandlung der Gärgutträger 7 sind diese anschließend keimfrei und bereit für eine erneute Befüllung mit Teigportionen sind.

Eine besonders bevorzugte Ausführungsform des Gärschranks sieht vor, dass der Außenrahmen 10 und die Rahmenprofile frei von Bohrungen und anderen Löchern oder Ausnehmungen sind, sondern glatt ausgebildet sind. Dies Verhindert, dass sich Schmutz in Nischen oder Löchern ansammelt und dort Schimmelbildung oder Bakterien-Herde entstehen. Alle an dem Außenrahmen 10 befestigten Elemente sind dabei über Klemmverbindungen, die auf die Rahmenprofile aufgeklemmt werden oder durch Verschweißungen an dem Außenrahmen 10 befestigt.

## Patentansprüche

1. Gärschrank (100) zum Gären von Teig umfassend
- einen Außenrahmen (10), der in einem Winkel zur Schwerkraftrichtung verlaufende Rahmenprofile umfasst und einen Innenraum (30) des Gärschranks umschließt,
- eine Anzahl an am Außenrahmen (10) angeordneten Abdeckpaneelen (3, 4, 5),
- eine durch den Innenraum (30) des Gärschranks (100) verlaufende Fördervorrichtung (6), wobei die Fördervorrichtung (6) dazu ausgebildet ist, Teiglinge durch den Gärschrank (100) zu transportieren, und wobei die Fördervorrichtung (6) eine Anzahl von im Wesentlichen horizontal oder vertikal, insbesondere horizontal oder vertikal, angeordneten Transportebenen aufspannt,
wobei zumindest eine Profilfläche der Rahmenprofile des Außenrahmens (10), die in einem Winkel zur Schwerkraftrichtung verlaufen und unterhalb derjenigen Transportebene oder desjenigen Punktes der Transportebenen angeordnet sind, die bzw. der in Schwerkraftrichtung am obersten angeordnet ist, in Richtung des Innenraums (30) geneigt ist,
**dadurch gekennzeichnet, dass**
die Rahmenprofile als Formrohre (1) ausgebildet sind und einen quadratischen oder rechteckigen Querschnitt, vier Längskanten (12) und vier Seitenflächen (11) aufweisen, wobei der Außenrahmen (10) aus normal und parallel zur Schwerkraftrichtung verlaufenden Formrohren (1) gebildet ist und zwischen der Schwerkraftrichtung und den in Richtung des Innenraums (30) geneigten Seitenflächen (11) der normal zur Schwerkraftrichtung verlaufenden Formrohre (1) ein Winkel von kleiner als 60° eingeschlossen ist.

2. Gärschrank (100) nach Anspruch 1, **dadurch gekennzeichnet, dass** zwischen der Schwerkraftrichtung und den in Richtung des Innenraums (30) geneigten Seitenflächen (11) der normal zur Schwerkraftrichtung verlaufenden Formrohre (1) ein Winkel von kleiner als 45°, vorzugsweise kleiner als 30°, eingeschlossen ist.

3. Gärschrank (100) nach Anspruch 1 , **dadurch gekennzeichnet, dass** zwischen der Schwerkraftrichtung und den in Richtung des Innenraums (30) geneigten Seitenflächen (11) der normal zur Schwerkraftrichtung verlaufenden Formrohre (1) ein Winkel von 45° eingeschlossen ist und
dass an den von jeweils drei Formrohren (1) gebildeten Ecken des Gärschranks (100) bei den normal zur Schwerkraftrichtung verlaufenden Formrohren (1) jeweils eine der Flächendiagonalen der Querschnittsfläche parallel zur Schwerkraftrichtung und die jeweils andere Flächendiagonale normal zur Schwerkraftrichtung ausgerichtet ist und die Längskanten (12) der Formrohre (1) jeweils einen rechten Winkel miteinander einschließen.

4. Gärschrank (100) nach einem der vorangehenden Ansprüche, **dadurch gekennzeichnet, dass** der Außenrahmen (10) öffnungsfrei, insbesondere bohrungsfrei, ausgebildet ist und/oder, dass die Formrohre (1), des Außenrahmens (10) permanent stoffschlüssig, insbesondere durch Verschweißen, verbunden sind.

5. Gärschrank (100) nach einem der vorangehenden Ansprüche, **dadurch gekennzeichnet, dass** die Fördervorrichtung (6) als Platten- oder Band-Fördervorrichtung oder als Fördervorrichtung (6), an der Gärgutträger zur Aufnahme von Teiglingen angeordnet sind, ausgebildet ist.

6. Gärschrank (100) nach einem der vorangehenden Ansprüche, **dadurch gekennzeichnet, dass** die Abdeckpaneele (3, 4, 5), am Außenrahmen (10) derart angeordnet sind, dass eine geschlossene Außenverkleidung (2) um den Innenraum (30) des Gärschranks (100) mit einem Boden (21), einer dem Boden (21) gegenüberliegende Decke (22) und Seitenwänden (23) gebildet ist, sodass der Innenraum (30) des Gärschranks (100) flüssigkeitsdicht verschlossen ist.

7. Gärschrank (100) nach Anspruch 6, **dadurch gekennzeichnet, dass** die normal zur Schwerkraftrichtung verlaufenden Formrohre (1) des Außenrahmens (10) derart ausgerichtet sind, dass eine Längskante (12) in Schwerkraftrichtung weiter vom Boden (21) entfernt ist, als die übrigen drei Längskanten (12) des jeweiligen Formrohrs (1) und/oder
dass die parallel zur Schwerkraftrichtung verlaufenden Formrohre (1) derart ausgerichtet sind, dass jeweils zwei ihrer Seitenflächen (11) dem Innenraum (30) des Gärschranks (100) zugewandt sind.

8. Gärschrank (100) nach Anspruch 6 oder 7, **dadurch gekennzeichnet, dass** die die Decke (22) bildenden Decken-Abdeckpaneele (3) und die Formrohre (1) des Außenrahmens (10) reversibel formschlüssig verbunden sind, wobei die Decken-Abdeckpaneele (3) an zumindest eine, vorzugsweise zwei, Seitenflächen (11) jedes der Formrohre (1) angepasste Auflageflächen (31) aufweisen und wobei insbesondere vorgesehen ist, dass die Decken-Abdeckpaneele (3) jeweils auf zumindest zwei Formrohren (1) des Außenrahmens (10) aufliegen.

9. Gärschrank (100) nach einem der Ansprüche 6-8, **dadurch gekennzeichnet, dass** die den Boden (21) bildenden Boden-Abdeckpaneele (4) und die Formrohre (1) des Außenrahmens (10) reversibel formschlüssig verbunden sind, wobei die Boden-Abdeckpaneele (4) jeweils auf zumindest zwei Formrohren (1) des Außenrahmens (10) aufliegen oder in Führungsschienen (15) in oder unter den Außenrahmen (10) einschiebbar sind und wobei insbesondere vorgesehen ist, dass die Boden-Abdeckpaneele (4) zumindest eine in ihrer Neigung an eine Seitenfläche (11) der Formrohre (1) angepasste Außenwand (41), und vorzugsweise zumindest eine entlang einer Außenwand (41) verlaufende Griffleiste (42) aufweisen.

10. Gärschrank (100) nach einem der Ansprüche 6-9, **dadurch gekennzeichnet, dass** die die Seitenwände (23) bildenden Seiten-Abdeckpaneele (5) aufschwenkbar an den Formrohren (1) des Außenrahmens (10), insbesondere mittels in Bohrungen (13) in den, vorzugsweise normal zur Schwerkraftrichtung verlaufenden, Formrohren (1) einsteckbaren oder auf an den Formrohren angeschweißten Aufhängungen (55) befestigten Scharnieren (51), angeordnet sind.

11. Gärschrank (100) nach Anspruch 9 oder 10, **dadurch gekennzeichnet, dass** die Seiten-Abdeckpaneele (5) aus einer Schließstellung, in der die Seiten-Abdeckpaneele (5) an zumindest zwei Formrohren (1) des Außenrahmens (10) aufliegen, verschwenkbar sind und dass an den Seiten-Abdeckpaneelen (5) jeweils zumindest ein Schalter (52) angeordnet ist, der dazu ausgebildet ist, ein Verschwenken der Seiten-Abdeckpaneele (5) aus der Schließstellung zu detektieren.

12. Gärschrank (100), nach einem der vorangehenden Ansprüche, **dadurch gekennzeichnet, dass** die Fördervorrichtung (6) ein Riemen- oder Kettengetriebe mit einem Riemen- oder Kettensystem (61) und einer Spannvorrichtung (62) zum selbstständigen Spannen des Riemen- oder Kettensystems (61) umfasst,
wobei die Spannvorrichtung (62) derart ausgebildet ist, dass bei Kräfteveränderung im Riemen- oder Kettensystem (61) die Riemenscheibe oder das Kettenrad (18) relativ zum Außenrahmen (10) verstellbar ist,
wobei insbesondere vorgesehen ist, dass die Spannvorrichtung (62)
- zumindest einen Spannzylinder (16) mit einem darin beweglichen Kolben (17) umfasst, wobei der Kolben (17) derart zwischen dem Außenrahmen (10) und einer Riemenscheibe oder einem Kettenrad (18) des Riemen- oder Kettensystems (61) angeordnet ist, oder
- als Feder, insbesondere Gasdruckfeder, ausgebildet ist.

13. Gärschrank (100) nach einem der vorangehenden Ansprüche, **dadurch gekennzeichnet, dass** an den Formrohren (1) des Außenrahmens (10) Halterungen (14) für Antriebselemente der Fördervorrichtung (6) reversibel formschlüssig angeordnet, insbesondere verschraubt, sind.

14. Gärschrank (100) nach einem der vorangehenden Ansprüche, **dadurch gekennzeichnet, dass** eine Reinigungsvorrichtung zur Reinigung des Innenraums (30) vorgesehen ist, die zumindest eine, insbesondere am Außenrahmen (10) angeordnete, Wasserabgabevorrichtung und/oder zumindest eine Ablauföffnung umfasst, wobei die Ablauföffnung in den den Boden (21) der Außenverkleidung (20) bildenden Boden-Abdeckpaneelen (4) ausgebildet ist.

15. Gärschrank (100) nach einem der vorangehenden Ansprüche, **dadurch gekennzeichnet, dass** eine Reinigungsstation zur Reinigung der Gärgutträger (7) vorgesehen ist, wobei die Reinigungsstation im Innenraum (30) des Gärschranks (100) oder in einem an den Innenraum (30) angeschlossenen Zusatzgehäuse angeordnet ist wobei die Reinigungsstation zumindest eine Absaugvorrichtung und/oder Bürsten und/oder zumindest eine Wasserabgabevorrichtung umfasst und wobei insbesondere vorgesehen ist, dass die Fördervorrichtung (6) dazu ausgebildet ist, die Gärgutträger (7) durch die Reinigungsstation zu transportieren.

16. Gärschrank (100) nach Anspruch 15, **dadurch gekennzeichnet, dass** die Reinigungsstation eine Trocknungsvorrichtung zur Trocknung der Gärgutträger (7), insbesondere mittels Infrarotstrahlung, und/oder eine Entkeimungsvorrichtung zur Desinfektion der Gärgutträger (7) umfasst, wobei insbesondere vorgesehen ist, dass die Entkeimungsvorrichtung zumindest eine Vorrichtung zum Auftragen von Wasserstoffperoxid auf die Gärgutträger (7) und/oder zumindest ein Beleuchtungsmittel zur Bestrahlung der Gärgutträger (7) mit ultraviolettem Licht umfasst.

## Claims

1. Proofing chamber (100) for proofing dough, comprising
- an outer frame (10) which comprises profiled frame sections running at an angle to the gravitational direction and which encloses the interior (30) of the proofing chamber,
- a number of cover panels (3, 4, 5) which are arranged on the outer frame (10),
- a conveyor device (6) which runs through the interior (30) of the proofing chamber (100), wherein the conveyor device (6) is designed to transport dough pieces through the proofing chamber (100), and wherein the conveyor device (6) spans a number of transport planes arranged substantially horizontally or vertically, in particular horizontally or vertically,
wherein at least one profiled surface of the profiled frame sections of the outer frame (10) which run at an angle to the gravitational direction and are arranged below the transport plane or the point of the transport plane arranged at the top in the gravitational direction is inclined in the direction of the interior (30),
**characterised in that**
the profiled frame sections are designed as shaping tubes (1) and have a square or rectangular cross-section, four lengthwise edges (12) and four lateral surfaces (11), wherein the outer frame (10) is formed of shaping tubes (1) running perpendicular and parallel to the gravitational direction and, and between the gravitational direction and the lateral surfaces (11) of the shaping tubes (1) inclined in the direction of the interior (30) and running perpendicular to the gravitational direction, an angle of less than 60° is enclosed.

2. Proofing chamber (100) according to claim 1, **characterised in that** between the gravitational direction and the lateral surfaces (11) of the shaping tubes (1) inclined in the direction of the interior (30) and running perpendicular to the gravitational direction, an angle of less than 45°, preferably less than 30°, is enclosed.

3. Proofing chamber (100) according to claim 1, **characterised in that** between the gravitational direction and the lateral surfaces (11) of the shaping tubes (1) inclined in the direction of the interior (30) and running perpendicular to the gravitational direction, an angle of 45° is enclosed and
**in that**, at the corners of the proofing chamber (100) formed in each case by three shaping tubes (1), in the shaping tubes (1) running perpendicular to the gravitational direction, in each case one of the face diagonals of the cross-section surface is directed parallel to the gravitational direction and the respective other diagonal plane is directed perpendicular to the gravitational direction, and the lengthwise edges (12) of the shaping tubes (1) each form a right angle with one another.

4. Proofing chamber (100) according to any one of the preceding claims, **characterised in that** the outer frame (10) is free of openings, in particular free of bore holes, and/or **in that** in the shaping tubes (1) of the outer frame (10) are permanently integrally joined, in particular by soldering.

5. Proofing chamber (100) according to any one of the preceding claims, **characterised in that** the conveyor device (6) is configured as a plate or belt conveyor device or as a conveyor device (6) on which proofing material carriers for receiving dough pieces are arranged.

6. Proofing chamber (100) according to any one of the preceding claims, **characterised in that** the cover panels (3, 4, 5) are arranged on the outer frame (10) in such a way that a closed outer lining (2) is formed around the interior (30) of the proofing chamber (100) and has a base (21), a ceiling (22) opposite the base (21) and side walls (23), such that the interior (30) of the proofing chamber (100) is sealed in a liquid-tight manner.

7. Proofing chamber (100) according to claim 6, **characterised in that** the shaping tubes (1) of the outer frame (10) running perpendicular to the gravitational direction are oriented in such a way that one lengthwise edge (12) is further away from the base (21) in the gravitational direction than the other three lengthwise edges (12) of the respective shaping tube (1) and/or
**in that** the shaping tubes (1) running parallel to the gravitational direction are oriented in such a way that in each case two of their lateral surfaces (11) face the interior (30) of the proofing chamber (100).

8. Proofing chamber (100) according to claim 6 or 7, **characterised in that** the ceiling cover panels (3) forming the ceiling (22), and the shaping tubes (1) of the outer frame (10) are reversibly interlockingly connected, wherein the ceiling cover panels (3) have bearing surfaces (31) adapted to at least one, preferably two, lateral surfaces (11) of each of the shaping tubes (1) and wherein in particular it is provided that the ceiling cover panels (3) each bear on at least two shaping tubes (1) of the outer frame (10).

9. Proofing chamber (100) according to any one of claims 6-8, **characterised in that** the base cover panels (4) forming the base (21) and the shaping tubes (1) of the outer frame (10) are reversibly interlockingly connected, wherein the base cover panels (4) each bear on at least two shaping tubes (1) of the outer frame (10) or can be inserted into guide rails (15) in or under the outer frame (10), and wherein in particular it is provided that the base cover panels (4) have at least one outer wall (41) whose inclination is adapted to a lateral surface (11) of the shaping tubes (1), and preferably at least one handle strip (42) running along an outer wall (41).

10. Proofing chamber (100) according to any one of claims 6-9, **characterised in that** the side cover panels (5) forming the side walls (23) are arranged such that they can be pivoted open on the shaping tubes (1) of the outer frame (10), in particular by means of hinges (51) which can be inserted into bore holes (13) in the shaping tubes (1), which preferably run perpendicular to the gravitational direction or are fastened on suspensions (55) welded to the shaping tubes.

11. Proofing chamber (100) according to claim 9 or 10, **characterised in that** the side cover panels (5) can be pivoted out of a closed position in which the side cover panels (5) bear on at least two shaping tubes (1) of the outer frame (10), and **in that** at least one switch (52) is arranged on each of the side cover panels (5), which switch is designed to detect pivoting of the side cover panels (5) out of the closed position.

12. Proofing chamber (100) according to any one of the preceding claims, **characterised in that** the conveyor device (6) comprises a belt or chain gear with a belt or chain system (61) and a tensioning device (62) for independent tensioning of the belt or chain system (61),
wherein the tensioning device (62) is designed in such a way that the belt pulley or the chain sprocket (18) is adjustable relative to the outer frame (10) in the event of a change in force in the belt or chain system (61),
wherein it is provided in particular that the tensioning device (62)
- comprises at least one tensioning cylinder (16) with a piston (17) movable therein, wherein the piston (17) is arranged between the outer frame (10) and a belt pulley or chain sprocket (18) of the belt or chain system (61), or
- is designed as a spring, in particular a gas
strut.

13. Proofing chamber (100) according to any one of the preceding claims, **characterised in that** brackets (14) for drive elements of the conveyor device (6) are arranged in a reversible interlocking manner, in particular are screwed, on the shaping tubes (1) of the outer frame (10).

14. Proofing chamber (100) according to any one of the preceding claims, **characterised in that** a cleaning device for cleaning the interior (30) is provided, and comprises at least one water dispensing device, which is in particular arranged on the outer frame (10), and/or comprises at least one drainage opening, wherein the drainage opening is formed in the base cover panels (4) forming the base (21) of the outer lining (20).

15. Proofing chamber (100) according to any one of the preceding claims, **characterised in that** a cleaning station is provided for cleaning the proofing material carriers (7), wherein the cleaning station is arranged in the interior (30) of the proofing chamber (100) or in an additional housing connected to the interior (30), wherein the cleaning station comprises at least one suction device and/or brushes and/or at least one water dispensing device, and wherein it is provided in particular that the conveying device (6) is designed to transport the proofing material carriers (7) through the cleaning station.

16. Proofing chamber (100) according to claim 15, **characterised in that** the cleaning station comprises a drying device for drying the proofing material carriers (7), in particular by means of infrared radiation, and/or a disinfecting device for disinfecting the proofing material carriers (7), wherein it is provided in particular that the disinfecting device comprises at least one device for applying hydrogen peroxide to the proofing material carriers (7) and/or at least one illuminating means for irradiating the proofing material carriers (7) with ultraviolet light.

## Revendications

1. Chambre de fermentation (100) pour la fermentation de pâte comprenant
- un cadre extérieur (10) qui comprend des profilés de cadre s'étendant selon un angle par rapport à la direction de la gravité et qui entoure un espace intérieur (30) de la chambre de fermentation,
- un certain nombre de panneaux de recouvrement (3, 4, 5) agencés sur le cadre extérieur (10),
- un dispositif de transport (6) s'étendant à travers l'espace intérieur (30) de la chambre de fermentation (100), dans laquelle le dispositif de transport (6) est conçu pour transporter des pâtons à travers la chambre de fermentation (100), et dans laquelle le dispositif de transport (6) déploie un certain nombre de plans de transport agencés sensiblement horizontalement ou verticalement, en particulier horizontalement ou verticalement,
dans laquelle au moins une surface profilée des profilés de cadre du cadre extérieur (10), qui s'étendent selon un angle par rapport à la direction de la gravité et qui sont agencés au-dessous du plan de transport ou du point des plans de transport qui est agencé le plus haut dans la direction de la gravité, est inclinée en direction de l'espace intérieur (30),
**caractérisée en ce que**
les profilés de cadre sont conçus sous forme de tubes de formage (1) et présentent une section transversale carrée ou rectangulaire, quatre arêtes longitudinales (12) et quatre surfaces latérales (11), dans laquelle le cadre extérieur (10) est formé de tubes de formage (1) s'étendant perpendiculairement et parallèlement à la direction de la gravité et un angle inférieur à 60° est inclus entre la direction de la gravité et les surfaces latérales (11) inclinées en direction de l'espace intérieur (30) des tubes de formage (1) s'étendant perpendiculairement à la direction de la gravité.

2. Chambre de fermentation (100) selon la revendication 1, **caractérisée en ce qu'**un angle inférieur à 45°, de préférence inférieur à 30°, est inclus entre la direction de la gravité et les surfaces latérales (11) inclinées en direction de l'espace intérieur (30) des tubes de formage (1) s'étendant perpendiculairement à la direction de la gravité.

3. Chambre de fermentation (100) selon la revendication 1, **caractérisée en ce qu'**un angle de 45° est inclus entre la direction de la gravité et les surfaces latérales (11) inclinées en direction de l'espace intérieur (30) des tubes de formage (1) s'étendant perpendiculairement à la direction de la gravité, et
**en ce que**, au niveau des coins de la chambre de fermentation (100) formés respectivement par trois tubes de formage (1), pour les tubes de formage (1) s'étendant perpendiculairement à la direction de la gravité, respectivement l'une des diagonales de surface de la surface de section transversale est orientée parallèlement à la direction de la gravité et l'autre diagonale de surface respective est orientée perpendiculairement à la direction de la gravité, et les arêtes longitudinales (12) des tubes de formage (1) incluent respectivement un angle droit entre elles.

4. Chambre de fermentation (100) selon l'une quelconque des revendications précédentes, **caractérisée en ce que** le cadre extérieur (10) est conçu sans ouverture, en particulier sans perçage, et/ou **en ce que** les tubes de formage (1) du cadre extérieur (10) sont reliés en permanence par liaison de matière, en particulier par soudage.

5. Chambre de fermentation (100) selon l'une quelconque des revendications précédentes, **caractérisée en ce que** le dispositif de transport (6) est conçu comme un dispositif de transport à plaques ou à bande ou comme un dispositif de transport (6) sur lequel sont agencés des supports de produits à fermenter destinés à recevoir des pâtons.

6. Chambre de fermentation (100) selon l'une quelconque des revendications précédentes, **caractérisée en ce que** les panneaux de recouvrement (3, 4, 5) sont agencés sur le cadre extérieur (10) de sorte qu'un revêtement extérieur fermé (2) est formé autour de l'espace intérieur (30) de la chambre de fermentation (100) avec un fond (21), un plafond (22) opposé au fond (21) et des parois latérales (23), de sorte que l'espace intérieur (30) de la chambre de fermentation (100) est fermé de manière étanche aux liquides.

7. Chambre de fermentation (100) selon la revendication 6, **caractérisée en ce que** les tubes de formage (1) du cadre extérieur (10) s'étendant perpendiculairement à la direction de la gravité sont orientés de sorte qu'une arête longitudinale (12) est plus éloignée du fond (21) dans la direction de la gravité que les trois autres arêtes longitudinales (12) du tube de formage (1) respectif et/ou
**en ce que** les tubes de formage (1) s'étendant parallèlement à la direction de la gravité sont orientés de sorte que respectivement deux de leurs surfaces latérales (11) sont tournées vers l'espace intérieur (30) de la chambre de fermentation (100).

8. Chambre de fermentation (100) selon la revendication 6 ou 7, **caractérisée en ce que** les panneaux de recouvrement de plafond (3) formant le plafond (22) et les tubes de formage (1) du cadre extérieur (10) sont reliés de manière réversible par complémentarité de forme, dans laquelle les panneaux de recouvrement de plafond (3) sont fixés sur au moins une, de préférence deux, surfaces d'appui (31) adaptées à chacun des tubes de formage (1), et dans laquelle il est notamment prévu que les panneaux de recouvrement de plafond (3) reposent chacun sur au moins deux tubes de formage (1) du cadre extérieur (10).

9. Chambre de fermentation (100) selon l'une quelconque des revendications 6-8, **caractérisée en ce que** les panneaux de recouvrement de fond (4) formant le fond (21) et les tubes de formage (1) du cadre extérieur (10) sont reliés de manière réversible par complémentarité de forme, dans laquelle les panneaux de recouvrement de fond (4) reposent chacun sur au moins deux tubes de formage (1) du cadre extérieur (10) ou peuvent être insérés dans des rails de guidage (15) dans ou sous le cadre extérieur (10) et dans laquelle il est en particulier prévu que les panneaux de recouvrement de fond (4) présentent au moins une paroi extérieure (41) dont l'inclinaison est adaptée à une surface latérale (11) des tubes de formage (1), et de préférence au moins une barre de préhension (42) s'étendant le long d'une paroi extérieure (41).

10. Chambre de fermentation (100) selon l'une quelconque des revendications 6-9, **caractérisée en ce que** les panneaux de recouvrement latéraux (5) formant les parois latérales (23) sont agencés de manière à pouvoir s'ouvrir par pivotement sur les tubes de formage (1) du cadre extérieur (10), en particulier au moyen de charnières (51) pouvant être insérées dans des perçages (13) dans les tubes de formage (1), de préférence perpendiculairement par rapport à la direction de la gravité, ou fixées sur des suspensions (55) soudées sur les tubes de formage.

11. Chambre de fermentation (100) selon la revendication 9 ou 10, **caractérisée en ce que** les panneaux de recouvrement latéraux (5) peuvent pivoter à partir d'une position de fermeture, dans laquelle les panneaux de recouvrement latéraux (5) reposent sur au moins deux tubes de formage (1) du cadre extérieur (10), et **en ce que** sur les panneaux de recouvrement latéraux (5) est agencé respectivement au moins un commutateur (52), lequel est conçu pour détecter un pivotement des panneaux de recouvrement latéraux (5) à partir de la position de fermeture.

12. Chambre de fermentation (100), selon l'une quelconque des revendications précédentes, **caractérisée en ce que** le dispositif de transport (6) comprend une transmission à courroie ou à chaîne avec un système de courroie ou de chaîne (61) et un dispositif de tension (62) pour tendre de manière autonome le système de courroie ou de chaîne (61),
dans laquelle le dispositif de tension (62) est conçu de sorte que, en cas de modification des forces dans le système de courroie ou de chaîne (61), la poulie ou le pignon à chaîne (18) est réglable par rapport au cadre extérieur (10),
dans laquelle il est notamment prévu que le dispositif de serrage (62)
- comprend au moins un cylindre de tension (16) avec un piston (17) mobile dans celui-ci, dans laquelle le piston (17) est agencé entre le cadre extérieur (10) et une poulie ou un pignon à chaîne (18) du système de poulie ou de chaîne (61), ou
- qu'il est conçu comme un ressort, en particulier un ressort à gaz.

13. Chambre de fermentation (100) selon l'une quelconque des revendications précédentes, **caractérisée en ce que** des supports (14) pour des éléments d'entraînement du dispositif de transport (6) sont agencés de manière réversible par complémentarité de forme, en particulier vissés, sur les tubes de formage (1) du cadre extérieur (10).

14. Chambre de fermentation (100) selon l'une quelconque des revendications précédentes, **caractérisée en ce qu'**un dispositif de nettoyage est prévu pour le nettoyage de l'espace intérieur (30), lequel comprend au moins un dispositif de distribution d'eau, en particulier agencé sur le cadre extérieur (10), et/ou au moins une ouverture d'écoulement, dans laquelle l'ouverture d'écoulement est réalisée dans les panneaux de recouvrement de fond (4) formant le fond (21) du revêtement extérieur (20).

15. Chambre de fermentation (100) selon l'une quelconque des revendications précédentes, **caractérisée en ce qu'**une station de nettoyage est prévue pour le nettoyage des supports de produits à fermenter (7), dans laquelle la station de nettoyage est agencée dans l'espace intérieur (30) de la chambre de fermentation (100) ou dans un boîtier supplémentaire raccordé à l'espace intérieur (30), dans laquelle la station de nettoyage comprend au moins un dispositif d'aspiration et/ou des brosses et/ou au moins un dispositif de distribution d'eau et dans laquelle il est prévu en particulier que le dispositif de transport (6) est conçu pour transporter les supports de produits à fermenter (7) à travers la station de nettoyage.

16. Chambre de fermentation (100) selon la revendication 15, **caractérisée en ce que** la station de nettoyage comprend un dispositif de séchage pour sécher les supports de produits à fermenter (7), en particulier au moyen d'un rayonnement infrarouge, et/ou un dispositif de désinfection pour désinfecter les supports de produits à fermenter (7), dans laquelle il est en particulier prévu que le dispositif de désinfection comprend au moins un dispositif pour appliquer du peroxyde d'hydrogène sur les supports de produits à fermenter (7) et/ou au moins un moyen d'éclairage pour irradier les supports de produits à fermenter (7) avec une lumière ultraviolette.
